# EUROPÄISCHE PATENTSCHRIFT

(11) **EP 2 121 071 B1**
(45) Veröffentlichungstag und Bekanntmachung des Hinweises auf die Patenterteilung: **12.04.2017**
(21) Anmeldenummer: 08707315.1
(22) Anmeldetag: 26.01.2008
(51) Int. Cl.: A61M 1/02, A61M 1/36

(54) **VERFAHREN UND VORRICHTUNG ZUR VERARBEITUNG VON BLUT UND BEUTELSYSTEM FÜR EINE BLUTVERARBEITUNGSVORRICHTUNG**
METHOD AND DEVICE FOR PROCESSING BLOOD AND BAG SYSTEM FOR A BLOOD PROCESSING DEVICE
PROCÉDÉ ET DISPOSITIF DE TRAITEMENT DE SANG ET SYSTÈME DE POCHES POUR UN PROCÉDÉ DE TRAITEMENT DE SANG

(30) Priorität: 31.01.2007 DE 102007004722
(43) Veröffentlichungstag der Anmeldung: 25.11.2009
(73) Patentinhaber: Fresenius HemoCare Deutschland GmbH, 61352 Bad Homburg v.d.H. (DE)
(72) Erfinder: NEUMANN, Hans-Jürgen, 66606 St. Wendel (DE)
(74) Vertreter: Kusche, Robert
(86) Internationale Anmeldenummer: PCT/EP2008/000609
(87) Internationale Veröffentlichungsnummer: WO 2008/092610

(56) Entgegenhaltungen:
- WO-A-01/74158
- WO-A-99/16480
- DE-A1- 3 012 227
- US-A- 6 053 885

## Beschreibung

Die Erfindung bezieht sich auf ein Verfahren und eine Vorrichtung zur Verarbeitung von Vollblut, das durch Zentrifugieren in die Blutkomponenten getrennt ist, oder von mindestens einer durch Zentrifugieren abgetrennten Blutkomponente. Darüber hinaus betrifft die Erfindung ein Beutelsystem für eine Blutverarbeitungsvorrichtung.

Bei den allgemein bekannten Verfahren zur Isolation von Blutbestandteilen aus Vollblut wird das Vollblut in einer Kammer zentrifugiert. Auf Grund der unterschiedlichen Sedimentationsgeschwindigkeiten der Blutkomponenten trennt sich das Vollblut beim Zentrifugieren im Wesentlichen in drei Phasen unterschiedlicher Dichte. Die Schicht mit der geringsten Dichte besteht aus Blutplasma (PLS), die Schicht mit der mittleren Dichte enthält den sogenannten Buffy Coat (BC), und die Schicht mit der größten Dichte enthält die Erythrozyten, die auch als Red Cell Concentrate (RCC) bezeichnet werden.

Die einzelnen Blutkomponenten sollen in jeweils eigene Beutel überführt werden. Um die einzelnen Schichten in der Zentrifugationskammer voneinander zu trennen, ist ein weiterer Verfahrensschritt erforderlich. Es sind Vorrichtungen bekannt, mit denen die beiden Verfahrensschritte des Zentrifugierens des Vollblutes und des Abtrennens der einzelnen Blutkomponenten automatisiert innerhalb einer Blutzentrifuge durchgeführt werden können. Es sind aber auch Verfahren bekannt, bei denen das bereits in seine Komponenten getrennte Vollblut nach dem Zentrifugieren in einer separaten Vorrichtung verarbeitet wird, um die einzelnen Komponenten in jeweils eigene Beutel zu überführen. Bei diesem Transfer der Flüssigkeiten ist von Bedeutung, dass keine Kontamination des Blutes oder andere nachteilige Reaktionen, wie beispielsweise eine Hämolyse, auftreten. Daher finden im Allgemeinen als Disposable ausgebildete Beutelsysteme Verwendung, die in die Blutverarbeitungsvorrichtungen eingelegt werden.

Die bekannten Beutelsysteme verfügen über einen Primärbeutel zur Aufnahme von Vollblut, der auch als Vollblutbeutel bezeichnet wird. In dem Primärbeutel wird das in seine Komponenten getrennte Vollblut bereitgestellt. An den Primärbeutel sind verschiedene Komponentenbeutel über Schlauchleitungen angeschlossen, die auch als Transferbeutel bezeichnet werden. Es sind Beutelsysteme mit Komponentenbeuteln zur Aufnahme von Erythrozyten (RCC), Blutplasma (PLS) und Buffy Coat (BC) bekannt. Neben diesen Transferbeuteln verfügen die bekannten Beutelsysteme vielfach über einen weiteren Komponentenbeutel, in dem eine Additivlösung für eine der anderen Blutkomponenten, insbesondere für die Erythrozyten bereitgestellt wird. Zusätzlich findet man in den bekannten Beutelsystemen einen Leukozytenfilter, der das Erythrozytenkonzentrat für eine weitere medizinische Verwendung notwendigerweise von Leukozyten befreit.

Die Bereitstellung des Vollblutes und der einzelnen Blutkomponenten in Beuteln hat weiterhin den Vorteil, dass sich die Flüssigkeiten nicht nur durch Schwerkraft, sondern beispielsweise auch durch Ausüben von Druck auf die Beutel fördern lassen. Dabei kann der Druck von Hand oder mit einer Vorrichtung auf die Beutel ausgeübt werden.

Die US-A-4,767,541 beschreibt ein Beutelsystem zur Trennung von Vollblut in seine Blutkomponenten. Einer der Beutel dient zur Aufnahme einer Additivlösung, die nach dem Zentrifugieren und Abtrennen des Blutplasmas durch einen Filter in den Primärbeutel geleitet wird, um dort mit den verbliebenen Erythrozyten und Buffy Coat vermischt und durch den Filter zurück in den Komponentenbeutel überführt zu werden. Nachteilig ist, dass dabei der Buffy Coat in dem Filter verbleibt, wodurch der Buffy Coat verloren geht. Nachteilig ist auch, dass zum Überführen der Additivlösung und Vermischen der Lösung mit den Blutkomponenten mehrere Schritte erforderlich sind.

Aus der EP-A-0 686 403 A1 ist ein Beutelsystem bekannt, bei dem die Additivlösung in einem eigenen Beutel bereitgestellt wird. Die Additivlösung wird aus dem Additivlösungsbeutel in den Primärbeutel überführt, um mit dem verbliebenen Erythrozytenkonzentrat vermischt zu werden.

Auch aus der US-A-6,053 885 ist ein Beutelsystem bekannt, bei dem sich die Additivlösung in einem eigenen Beutel befindet. Neben dem Beutelsystem wird in der US-A-6,053,885 eine Vorrichtung zum Auspressen der Beutel beschrieben. Das in die Blutkomponenten getrennte Vollblut wird in einem Primärbeutel und die Additivlösung in einem Additivlösungsbeutel bereitgestellt, die zwischen zwei parallelen Anpressplatten ausgedrückt werden. Während das Blutplasma von oben aus dem Primärbeutel abgezogen werden kann, wird das Erythrozytenkonzentrat von unten abgeführt. Das Erythrozytenkonzentrat wird mit der Additivlösung vermischt, und die Mischung aus Erythrozytenkonzentrat und Additivlösung wird in einen Komponentenbeutel überführt. Dabei passiert die Mischung von Erythrozytenkonzentrat und Additivlösung einen Leukozytenfilter. Auch hier erweist sich als nachteilig, dass die Additivlösung in einem separaten Beutel bereitgestellt wird.

Die WO 2005/002644 A1 beschreibt wieder ein Verfahren zur Trennung von Blutkomponenten, bei dem die Additivlösung in einem separaten Beutel bereitgestellt und mit den nach dem Auspressen in dem Primärbeutel verbliebenden Erythrozyten vermischt wird.

Aus der WO 01/74158 A2 ist ein Beutelsystem bekannt, dass über einen Primärbeutel, einen Transferbeutel und einen Komponentenbeutel verfügt. Sämtliche Beutel weisen zwei Anschlüsse auf. Von einem der beiden Anschlüsse des Primärbeutels führt eine Schlauchleitung zu einem Y-Verbindungsstück, von dem eine Schlauchleitung abgeht, die zu einem Anschluss des Komponentenbeutels führt. Von dem anderen Anschluss des Komponentenbeutels geht eine Schlauchleitung ab, die zu einem Anschluss des Transferbeutels führt. In diese Schlauchleitung ist ein Filter geschaltet. Von dem anderen Anschluss des Transferbeutels geht eine Schlauchleitung ab, die wieder zum Y-Verbindungsstück führt. Das Beutelsystem wird wie Folgt gehandhabt. Zunächst wird aus dem Primärbeutel Plasma in den Transferbeutel überführt. Aus dem Komponentenbeutel wird dann eine Additivlösung dem Primärbeutel zugeführt, der als Lagerbeutel fungiert.

Die US 6 053 885 beschreibt ein Verfahren zur Verarbeitung von Vollblut, das durch Zentrifugieren in die Blutkomponenten getrennt ist, sowie ein Beutelsystem zur Verwendung bei dem bekannten Verfahren. Das Beutelsystem umfasst mehrere Beutel, von denen Schlauchleitungen zu einem Y-Verbindungsstück führen.

Der Erfindung liegt die Aufgabe zu Grunde, ein Verfahren zur Verarbeitung von durch Zentrifugieren in die Blutkomponenten getrennten Vollblut oder von mindestens einer durch Zentrifugieren abgetrennten Blutkomponente anzugeben, das eine separate Komponentenkammer zur Aufnahme einer Additivlösung überflüssig macht. Darüber hinaus ist eine Aufgabe der Erfindung, eine Blutbearbeitungsvorrichtung zu schaffen, die ohne eine separate Komponentenkammer zur Aufnahme einer Additivlösung auskommt, sowie ein Beutelsystem für eine Blutverarbeitungsvorrichtung bereitzustellen.

Die Lösung dieser Aufgaben erfolgt erfindungsgemäß mit den Merkmalen der Patentansprüche 1, 8 und 17. Vorteilhafte Ausführungsformen der Erfindung sind Gegenstand der Unteransprüche.

Das erfindungsgemäße Verfahren und die erfindungsgemäße Vorrichtung zeichnen sich dadurch aus, dass die Additivlösung zusammen mit mindestens einer Blutkomponente in einer Komponentenkammer bereitgestellt wird. Daher ist eine separate Komponentenkammer für die Additivlösung nicht erforderlich. Die Komponentenkammer zur Aufnahme der Additivlösung und der Blutkomponente ist in ein erstes und ein zweites Kompartiment mit variablem Volumen getrennt. Während das erste Kompartiment die Blutkomponente aufnimmt, nimmt das zweite Kompartiment die Additivlösung auf.

Beim Fördern der Flüssigkeiten wird das Volumen des ersten Kompartiments vergrößert, während das Volumen des zweiten Kompartiments verkleinert wird. Die Additivlösung wird somit aus dem zweiten Kompartiment gefördert, dessen Volumen sich verringert, und mit der Blutkomponente vermischt. Gleichzeitig wird die Mischung aus der Blutkomponente und der Additivlösung in das erste Kompartiment überführt, dessen Volumen sich vergrößert. Folglich erlauben das erfindungsgemäße Verfahren und die erfindungsgemäße Vorrichtung das Mischen der Additivlösung und der Blutkomponente in einem kontinuierlichen einstufigen Prozess. Dabei kann die Flüssigkeit allein aufgrund der Volumenvergrößerung oder -verringerung der Kammer oder auch mittels zusätzlicher Pumpen gefördert werden.

Für das erfindungsgemäße Verfahren und die erfindungsgemäße Vorrichtung ist unerheblich, ob in der Primärkammer Vollblut bereitgestellt wird, das durch Zentrifugieren in seine Blutkomponenten getrennt ist, oder sich in der Primärkammer nur eine oder mehrere Blutkomponenten befinden. Entscheidend ist, dass mindestens eine Blutkomponente aus der Primärkammer in die Komponentenkammer überführt wird, wobei die Blutkomponente beim Überführen in die Komponentenkammer mit der zuvor in der Komponentenkammer befindlichen Additivlösung vermischt wird.

Das Volumen des ersten Kompartiments wird um einen Betrag vergrößert, der derart bemessen ist, dass das erste Kompartiment neben der Additivlösung auch die mindestens eine Blutkomponente aufnehmen kann. Wenn sich das Volumen des ersten Kompartiments pro Zeiteinheit nicht um einen zusätzlichen Betrag vergrößert, wie das Volumen des zweiten Kompartiments abnimmt, muss sichergestellt sein, dass zu Beginn des Mischvorgangs in dem ersten Kompartiments bereits ein Volumen vorhanden ist, das ausreichend ist, um die Menge an der Blutkomponente aufzunehmen. Dies ist auf jeden Fall dann sichergestellt, wenn der Beutel derart flexibel ausgebildet ist, dass er sich um das zusätzliche Volumen ausdehnen kann.

Beim Überführen der Mischung aus Blutkomponente und Additivlösung wird die Mischung vorzugsweise gefiltert. Für den Fall, dass es sich bei der Blutkomponente um ein Erythrozytenkonzentrat handelt, wird die Mischung aus Erythrozytenkonzentrat und Additivlösung vorzugsweise in einem Leukozytenfilter gefiltert, um ein von Leukozyten befreites Erythrozytenkonzentrat gewinnen zu können.

Eine bevorzugte Ausführungsform der Erfindung sieht vor, dass die Primärkammer und/oder die Komponentenkammer als Beutel ausgebildet sind, wobei die Beutel ein vorzugsweise zur einmaligen Verwendung bestimmtes Beutelsystem bilden, dass in die Blutverarbeitungsvorrichtung eingelegt werden kann. Bei der Verwendung eines Beutelsystems können die Flüssigkeiten in bekannter Weise dadurch gefördert werden, dass auf die Beutel Druck ausgeübt wird.

Der Beutel zur Aufnahme der Blutkomponente und der Additivlösung wird vorteilhafterweise dadurch in das erste und zweite Kompartiment geteilt, in dem der Beutel entlang des das erste vom zweiten Kompartiment trennenden Bereichs zusammengedrückt wird. Dabei kann das Fassungsvermögen (Volumen) des ersten Kompartiments dadurch vergrößert werden, in dem der das erste von dem zweiten Kompartiment trennende Bereich entlang des Komponentenbeutels verschoben wird, wodurch gleichzeitig das Fassungsvermögen (Volumen) des zweiten Kompartiments verkleinert wird.

Die erfindungsgemäße Blutverarbeitungsvorrichtung sieht Mittel zum Trennen der Komponentenkammer in das erste und zweite Kompartiment vor, die derart ausgebildet sind, dass das Volumen des ersten Kompartiment vergrößert und das Volumen des zweiten Kompartiments verkleinert werden kann.

Eine bevorzugte Ausführungsform der erfindungsgemäßen Blutverarbeitungsvorrichtung sieht vor, dass die Mittel zum Trennen der Komponentenkammer in das erste und zweite Kompartiment eine ebene ortsfeste Anpressplatte und einen entlang einer Linie einen Anpressdruck ausübenden Anpresskörper aufweisen, der in einer zu der Ebene der Anpressplatte parallelen Ebene geführt ist. Der Anpresskörper und die Anpressplatte sind unter Zwischenlage des Komponentenbeutels derart im Abstand zueinander angeordnet, dass der Komponentenbeutel im Bereich der Anlagefläche bzw. Anlagelinie dicht verschlossen wird. Wenn der Anpresskörper verschoben wird, vergrößert sich das Volumen des einen Kompartiments, während sich das Volumen des anderen Kompartiments verringert.

Der entlang einer Linie einen Anpressdruck ausübende Anpresskörper kann unterschiedlich ausgebildet sein. Bei einer bevorzugten Ausführungsform ist der Anpresskörper ein drehbar gelagerter zylindrischer Körper, der entlang des Komponentenbeutels linear geführt ist, so dass der Anpresskörper eine Anpresskraft entlang einer Linie auf den Komponentenbeutel ausübt.

Das erfindungsgemäße Beutelsystem für die erfindungsgemäße Blutverarbeitungsvorrichtung kann neben dem Primärbeutel zur Aufnahme von Vollblut oder mindestens einer Blutkomponente und dem eine Additivlösung enthaltenen Komponentenbeutel noch weitere Komponentenbeutel umfassen, die zur Aufnahme weiterer Blutkomponenten, beispielsweise des Blutplasmas oder des Buffy Coats bestimmt sind.

Die einzelnen Komponentenbeutel sind vorzugsweise über Schlauchleitungen miteinander verbunden, so dass die Flüssigkeiten von dem einen in den anderen Beutel überführt werden können.

Grundsätzlich ist es unerheblich, ob der Einlass der Komponentenkammer in der Gebrauchslage oben und der Auslass der Komponentenkammer in der Gebrauchslage unten liegt. Eine derartige Anordnung des Ein- und Auslasses hat aber insofern den Vorteil, als sich auf Grund der Schwerkraft die Additivlösung zu Beginn des Verfahrens in dem unteren Bereich der Komponentenkammer befindet, während deren oberer Bereich leer ist. Folglich lässt sich die Additivlösung von unten aus der Komponentenkammer abführen, während die Mischung aus der Blutkomponente und der Additivlösung der Komponentenkammer von oben zugeführt werden kann. Es ist grundsätzlich aber auch möglich, Ein- und Auslass anders anzuordnen, da zu Beginn des Verfahrens sich in der Komponentenkammer ohnehin nur die Additivlösung befindet, so dass nicht die Möglichkeit des Vermischens von Additivlösung und Blutkomponente besteht, beispielsweise, wenn der Komponentenbeutel in die Blutverarbeitungsvorrichtung eingelegt wird. Später verhindert die Blutbearbeitungsvorrichtung durch die Aufteilung der Komponentenkammer in die beiden Kompartiments ohnehin, dass sich beide Flüssigkeiten in der Kammer miteinander vermischen.

Die Mittel zum Mischen der Blutkomponente und der Additivlösung können unterschiedlich ausgebildet sein, solange beide Flüssigkeiten zusammengeführt werden. Hierzu genügt grundsätzlich schon ein Y-Verbindungsstück. In der Praxis hat sich aber gezeigt, dass allein das Zusammenführen der Flüssigkeiten nicht ausreichend sein kann. Für diesen Fall können die Mittel zum Mischen über Verwirbelungen in den Flüssigkeiten erzeugende Elemente verfügen, die das Vermischen verbessern.

Im Folgenden werden Ausführungsbeispiele der Erfindung unter Bezugnahme auf die Zeichnungen näher erläutert.

Es zeigen:
- Fig. 1: Ein erstes Ausführungsbeispiel der erfindungsgemäßen Vorrichtung zur Verarbeitung von Vollblut in stark vereinfachter schematischer Darstellung,
- Fig. 2: eine Prinzipdarstellung eines ersten Ausführungsbeispiels einer Einrichtung zum Trennen des Komponentenbeutels der Blutverarbeitungsvorrichtung von Fig. 1 in zwei Kompartiments mit variablem Volumen und
- Fig. 3: ein zweites Ausführungsbeispiel des erfindungsgemäßen Beutelsystems zusammen mit der erfindungsgemäßen Blutverarbeitungsvorrichtung in stark vereinfachter schematischer Darstellung.

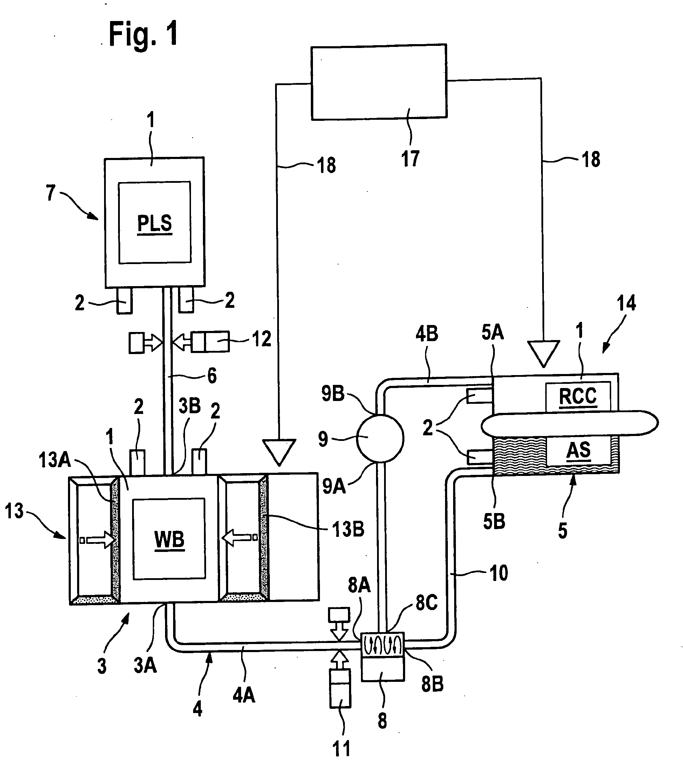

Fig. 1 zeigt ein erstes Ausführungsbeispiel der erfindungsgemäßen Vorrichtung zur Verarbeitung von Vollblut zusammen mit dem erfindungsgemäßen Beutelsystem, wobei die einzelnen Komponenten der Blutverarbeitungsvorrichtung und des Beutelsystems stark vereinfacht schematisch dargestellt sind.

Das erfindungsgemäße Beutelsystem des ersten Ausführungsbeispiels umfaßt insgesamt drei flexible Folienbeutel, in denen Flüssigkeiten bereitgestellt werden, bzw. in die Flüssigkeiten überführt werden. Die Folienbeutel können aus einem Folienschlauch gefertigt sein, der an seinen Enden verschweißt ist oder aus zwei übereinanderliegenden Folien, die an ihren Rändern miteinander verschweißt sind. Jeder Folienbeutel verfügt über zwei Anschlussstutzen zum Zuführen bzw. Abführen von Flüssigkeit, die dicht verschlossen sind. Derartige Folienbeutel mit Anschlussstutzen sind bekannt. Die Folienschläuche sämtlicher Beutel sind in Fig. 1 mit der Bezugsziffer 1 und deren Anschlussstutzen mit der Bezugsziffer 2 bezeichnet.

Das zuvor in einer separaten Blutzentrifuge in seine Blutkomponenten zentrifugierte Vollblut (WB) wird in einem Primärbeutel 3 bereitgestellt, der auch als Vollblutbeutel oder Spenderbeutel bezeichnet wird. Der Vollblutbeutel 3 enthält als oberste Schicht mit der geringsten Dichte das Blutplasma (PLS), als mittlere Schicht den sogenannten Buffy Coat (BC) mit einer mittleren Dichte und als unterste Schicht mit der größten Dichte die Erythrozyten, die auch als Red Cell Concentrate (RCC) bezeichnet werden.

Die oberste Schicht aus Blutplasma wird von dem Vollblutbeutel 3 von oben und die unterste Schicht von Erythrozyten von unten abgeführt. Hierzu geht von dem Auslass 3A am unteren Rand des Vollblutbeutels 3 eine flexible Schlauchleitung 4 ab, die zu einem ersten Komponentenbeutel 5 zur Aufnahme der Erythrozyten führt. Von dem Auslass 3B am oberen Rand des Vollblutbeutels 3 geht eine zweite Schlauchleitung 6 ab, die zu einem zweiten Komponentenbeutel 7 für Blutplasma führt.

Das Beutelsystem verfügt weiterhin über Mittel zum Vermischen des Erythrozytenkonzentrats mit einer Additivlösung, insbesondere einer Additivlösung für das Erythrozytenkonzentrat. Die Mischeinrichtung 8 ist nur schematisch dargestellt. Sie verfügt über einen ersten Einlass 8A, in den das Erythrozytenkonzentrat strömt, und einen zweiten Einlass 8B, in den die Additivlösung strömt sowie einen Auslass 8C, aus dem die Mischung aus Erythrozytenkonzentrat und Additivlösung strömt. Im einfachsten Fall handelt es sich bei der Mischeinrichtung um ein Y-Verbindungsstück, mit dem die Flüssigkeiten zusammengeführt werden. Vorzugsweise enthält die Mischeinrichtung aber Verwirbelungen erzeugende Elemente, so dass die Flüssigkeiten optimal vermischt werden.

Die von dem Vollblutbeutel 3 zu dem Erythrozytenbeutel 5 führende Schlauchleitung 4 weist einen ersten Abschnitt 4A auf, der von dem einen Auslass 3A des Vollblutbeutels 3 zu dem ersten Einlass 8A der Mischeinrichtung 8 führt, und einen zweiten Abschnitt 4B, der von dem Auslass 8C der Mischeinrichtung 8 zu dem Einlass 9A eines Filters 9 für die Erythrozytenfiltration, beispielsweise ein Leukozytenfilter zum Abtrennen der Leukozyten aus der Mischung von Erythrozytenkonzentrat und Additivlösung, führt und von dem Auslass 9B des Filters 9 abgeht und zu einem Einlass 5A des Erythrozytenbeutels 5 führt. Von einem Auslass 5B des Erythrozytenbeutels 5 geht eine weitere Schlauchleitung 10 für die Additivlösung ab, die zu dem zweiten Einlass 8B der Mischeinrichtung 8 führt.

Das Beutelsystem ist als ein zur einmaligen Verwendung bestimmtes Disposable ausgebildet, das in die Blutverarbeitungsvorrichtung eingelegt wird.

Die Blutbehandlungsvorrichtung verfügt über zwei Schlauchklemmen 11, 12, wobei der Schlauchleitungsabschnitt 4A des Beutelsystems 4 stromauf der Mischeinrichtung 8 in die eine Schlauchklemme 11 und die zu dem Plasmabeutel 7 führenden Schlauchleitung in die andere Schlauklemme 12 eingelegt wird. Darüber hinaus weist die Blutverarbeitungsvorrichtung eine erste Presseinrichtung 13, in die der Vollblutbeutel 3 eingelegt wird, und eine zweite Presseinrichtung 14 zum Einlegen des Erythrozytenbeutels 5 auf.

Die Presseinrichtung 13 für den Vollblutbeutel 3 verfügt über zwei im Abstand zueinander angeordnete Pressplatten 13A, 13B, zwischen denen der Vollblutbeutel 3 angeordnet wird. Wenn sich die beiden Pressplatten 13A, 13B aufeinander zu bewegen, wird der Beutel zusammengedrückt, wodurch Erythrozytenkonzentrat aus dem unteren Auslass 3A und Blutplasma aus dem oberen Auslass 3B des Vollblutbeutels 3 gefördert wird. Die Pressplatten 13A, 13B werden so lange aufeinander zu bewegt, bis sich in dem Vollblutbeutel nur noch der Buffy Coat befindet.

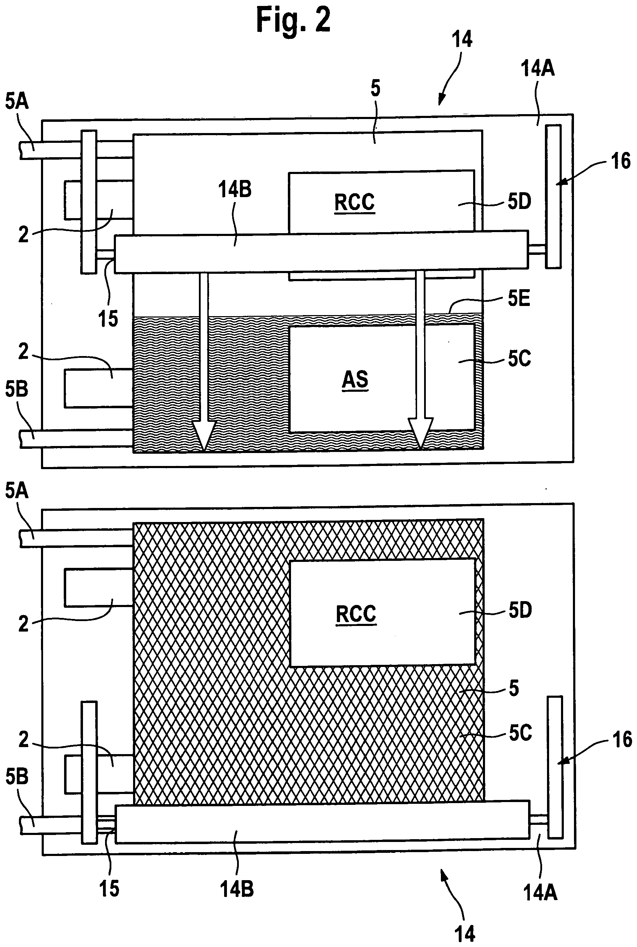

Nachfolgend wird die Presseinrichtung 14 für den Erythrozytenbeutel 1 unter Bezugnahme auf Fig. 2 im Einzelnen beschrieben. Fig. 2 zeigt die einzelnen Komponenten der Presseinrichtung 14 in schematischer Darstellung, wobei die Presseinrichtung in der oberen Bildhälfte zu Beginn und in der unteren Bildhälfte zum Ende des Auspressvorgangs dargestellt ist.

Die Presseinrichtung 14 verfügt über eine ortsfeste Anpressplatte 14A und einen Anpresskörper 14B, zwischen denen der Erythrozytenbeutel 5 angeordnet ist. Der Erythrozytenbeutel 5 ist derart angeordnet, dass dessen Einlass 5 A oben und dessen Auslass 5B unten liegt, wobei die Anpressplatte 14A in einer vertikalen Ebene liegt.

Der Anpresskörper 14B ist bei dem vorliegenden Ausführungsbeispiel ein drehbar gelagerter zylindrischer Körper. Diese Anpresswalze 14B erstreckt sich parallel zu der Anpressplatte 14A unter Zwischenlage des Erythrozytenbeutels 5.

Mit der Anpresswalze 14B wird entlang einer Linie 15, an der die Mantelfläche der Walze mit dem Beutel in Berührung kommt, eine Anpresskraft ausgeübt. Die Anpresswalze 14B ist entlang des Beutels 5 in Pfeilrichtung verschiebbar geführt, wobei die Walze auf dem Beutel abrollt. Für die translatorische Bewegung der Anpresswalze 14B ist eine Antriebseinheit 16 vorgesehen, die in Fig. 2 andeutungsweise dargestellt ist.
Für die Steuerung der Pressplatten beider Presseinrichtungen 13, 14 sowie der elektromagnetisch oder pneumatisch betätigbaren Schlauchklemmen 11, 12 weist die Blutverarbeitungsvorrichtung eine Steuereinheit 17 auf, die über nur andeutungsweise Steuerleitungen 18 mit den einzelnen Komponenten der Presseinrichtungen verbunden ist. Die Schlauchklemmen können aber auch nur manuell geöffnet oder geschlossen werden.

Die erfindungsgemäße Blutverarbeitungsvorrichtung arbeitet wie folgt. Während sich das Vollblut in dem Vollblutbeutel 3 befindet, wird die Additivlösung in dem Erythrozytenbeutel 5 bereitgestellt, der später zur Aufnahme des mit der Additivlösung versetzten Erythrozytenkonzentrats dient. Der Vollblutbeutel 3 wird in die Presseinrichtung 13 und der Erythrozytenbeutel 5 in die Presseinrichtung 14 eingelegt. Der Erythrozytenbeutel 5 wird dabei derart positioniert, dass dessen Einlass 5A oben und dessen Auslass 5B unten liegt, wobei die Additivlösung (AS) sich auf Grund der Schwerkraft in der unteren Beutelhälfte 5C befindet, während die obere Beutelhälfte 5D leer ist. Daraufhin wird die Anpresswalze 14B auf der oberen Beutelhälfte oberhalb des Flüssigkeitsspiegels 5E des nicht vollständig mit der Additivlösung befüllten Beutels positioniert. Die Anpresswalze 14B drückt dabei den Beutel 5 auf die Anpressplatte 14A, indem die Antriebseinheit 16 Druck auf die Walze ausübt. Grundsätzlich kann der Beutel auch liegend in eine Presseinrichtung eingelegt werden, deren Anpresswalze in horizontaler Richtung verfahrbar ist. Dies setzt aber voraus, dass die Walze in horizontaler Richtung über den Beutel von dem einen Rand zu dem anderen Rand derart verfahren wird, dass zwei Kompartiments gebildet werden, von denen die Additvlösung sich nur in einem der beiden Kompartiments befindet.

Die Schlauklemme 11 in dem zu der Mischeinrichtung 5 führenden Schlauchleitungsabschnitt 4A bleibt zunächst geschlossen, während die Schlauchklemme 12 in der zu dem Plasmabeutel 1 führenden Schlauchleitung 6 geöffnet wird.

Nunmehr wird die Presseinrichtung 13 für den Vollblutbeutel 3 in Betrieb gesetzt, wobei zunächst das Blutplasma in den Plasmabeutel 1 gepresst wird. Daraufhin wird die Schlauchklemme 12 geschlossen und die Schlauchklemme 11 geöffnet. Die Presseinrichtung 13 presst nunmehr das Erythrozytenkonzentrat aus dem Vollblutbeutel 3 aus. Bei geöffneten Schlauchklemmen 11, 12 können auch Blutplasma und Erythrozytenkonzentrat gleichzeitig aus dem Vollblutbeutel 3 ausgepresst werden, da der eine Auslass 3A am unteren und der andere Auslass 3B am oberen Rand des Beutels angeordnet ist.

Mit dem Betrieb der Presseinrichtung 13 startet die Steuereinheit 17 auch den Betrieb der Antriebseinheit 16 der Presseinrichtung 14 für den Erythrozytenbeutel 1, so dass sich die Anpresswalze 14B in Bewegung setzt. Die Anpresswalze 14B teilt den Erythrozytenbeutel 1 in ein erstes oberes Kompartiment 5D und ein zweites unteres Kompartiment 5C auf, während die Walze auf dem Beutel in Pfeilrichtung abrollt. Dabei vergrößert sich das Volumen des oberen Kompartiments 5D, während sich das Volumen des unteren Kompartiments 5C verringert. Dabei muss aber sichergestellt werden, dass das erste Kompartiment neben der Additivlösung auch die Blutkomponente aufnehmen kann.

Die sich in dem unteren Kompartiment 5C befindende Additivlösung wird durch die translatorische Bewegung der Anpresswalze 14B aus dem unteren Kompartiment 5C ausgedrückt, so dass in das obere Kompartiment 5D die Mischung aus Erythrozytenkonzentrat und Additivlösung zuströmen kann.

Während des Betriebs beider Presseinrichtungen 13 und 14 strömt das Erythrozytenkonzentrat RCC bei geöffneter Schlauchklemme 11 über den Schlauchleitungsabschnitt 4A der Schlauchleitung 4 zu dem ersten Einlass 8A der Mischeinrichtung 8, während die Additivlösung AS über die Schlauchleitung 10 zu dem zweiten Einlass 8B der Mischeinrichtung 8 strömt. Dort werden beide Flüssigkeiten miteinander vermischt. Die Mischung aus Erythrozytenkonzentrat und Additivlösung strömt aus dem Auslass 8C der Mischeinrichtung 8 über den Schlauchleitungsabschnitt 4B der Schlauchleitung 4 zu dem Einlass 5A des Erythrozytenbeutels 5. Dabei wird das mit der Additivlösung gemischte Erythrozytenkonzentrat in dem Filter 9 für die Erythrozytenfiltration von den Leukozyten befreit.

Während die Anpresswalze 14B von oben nach unten auf dem Beutel 5 abrollt, wird das untere Kompartiment 5C für die Additivlösung entleert und das obere Kompartiment 5D für die Mischung aus Erythrozytenkonzentrat und Additivlösung befüllt.

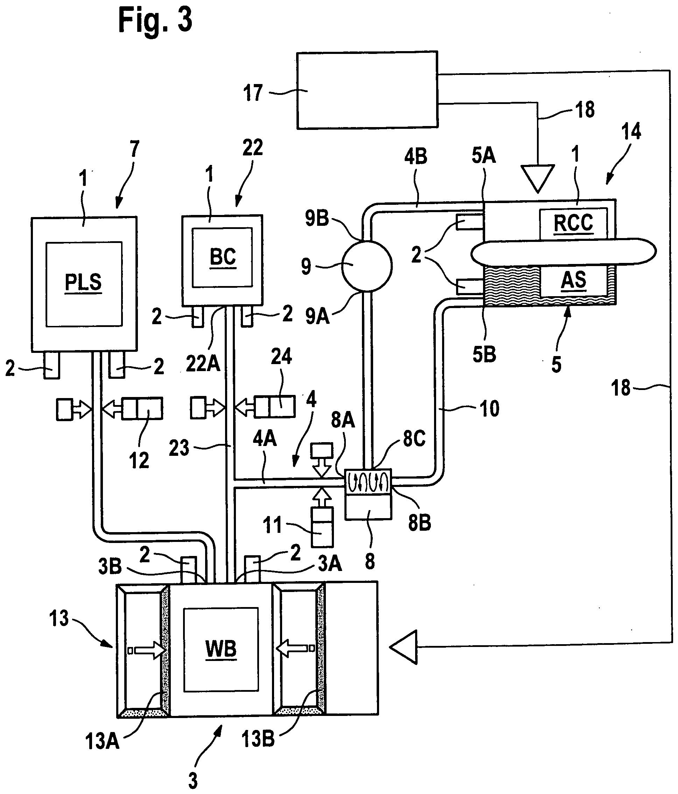

Die Steuereinheit 17 steuert den Vorschub der Anpresswalze 14B derart, dass der gesamte Vorrat an Additivlösung aus dem unteren Kompartiment 5C herausgepresst worden ist, wenn das obere Kompartiment 5D vollständig mit der Mischung aus Erythrozytenkonzentrat und Additivlösung befüllt ist. Die Anpresswalze 14B befindet sich dann in der in der unteren Bildhälfte von Fig. 3 gezeigten Position. Eventuelle Nichtlinearitäten in der Förderung der Additivlösung, die sich aus dem Vorschub der Anpresswalze 14B in Verbindung mit dem Fördervolumen ergeben können, werden vorzugsweise mit einer geregelten Vorschubsteuerung der Anpresswalze linearisiert. Hierzu kann der Vorschub der Anpresswalze derart gesteuert werden, dass sich eine vorbestimmte Förderrate für die Additivlösung einstellt.

Die Figuren 1 und 2 zeigen die Presseinrichtung zusammen mit dem Beutel nur in stark vereinfachter schematischer Darstellung, um das Funktionsprinzip zu erläutern. Durch unterschiedliche Beutelformen kann sichergestellt werden, dass sich pro Zeiteinheit das Volumen des ersten Kompartiments um einen größeren Betrag vergrößert wie sich das Volumen des zweiten Kompartiments verringert. Beispielsweise kann der Beutel derart ausgebildet sein, dass die Längsseiten nicht parallel zueinander verlaufen, sondern von oben nach unter schräg zusammenlaufen. Dann nimmt das Volumen in der ersten oberen Kammer um einen größeren Betrag pro Zeiteinheit zu wie das Volumen in der zweiten unteren Kammer abnimmt. Eine alternative Ausführungsform sieht beispielsweise einen Beutel vor, der derart flexibel ausgebildet ist, dass er sich um das zusätzlich erforderliche Volumen ausdehnen kann. Auch kann schon vor dem Mischvorgang in der einen Kammer ein zusätzliches Füllvolumen vorgesehen werden, dass ausreichend ist, um neben der Additivlösung auch die Blutkomponente noch aufnehmen zu können.

Der Vorteil der erfindungsgemäßen Blutverarbeitungsvorrichtung liegt darin, dass die Additivlösung in dem gleichen Beutel bereitgestellt werden kann, in dem auch die Mischung aus Erythrozytenkonzentrat und Additivlösung abgefüllt wird. Daher kann ein separater Transferbeutel eingespart werden. Hieraus ergibt sich nicht nur ein geringerer Aufwand, sondern das Verfahren wird auch beschleunigt, da der Vorgang des Förderns von Erythrozytenkonzentrat und des Vermischens desselben mit der Additivlösung in einem Schritt erfolgen kann.

Der Filter 9 sollte vor dem Überführen der Konzentrate gespült werden. Hierzu wird bei geschlossener Schlauchklemme 11 die Presseinrichtung 14 kurzzeitig in Betrieb gesetzt, so dass Additivlösung als Spüllösung gefördert wird. Die Additivlösung durchströmt die Mischeinrichtung 8 und benetzt den Filter, der somit für das weitere Verfahren vorbereitet ist.

Fig. 3 zeigt in vereinfachter schematischer Darstellung eine zweite Ausführungsform des Beutelsystems zusammen mit der Blutverarbeitungsvorrichtung, in die das Beutelsystem eingelegt ist. Das Beutelsystem von Fig. 3 unterscheidet sich von dem unter Bezugnahme auf Fig. 1 beschriebenen Beutelsystem dadurch, das ein weiterer Komponentenbeutel 22 für den Buffy Coat BC vorgesehen ist, dessen Einlass 22A über eine weitere Schlauchleitung 23 an den zu der Mischeinrichtung 8 führenden Schlauchleitungsabschnitt 4A stromauf der Schlauchklemme 11 angeschlossen ist, so dass der Komponentenbeutel über eine Schlauchleitung mit dem Vollblutbeutel verbunden ist. An der zu dem Komponentenbeutel 22 führenden Schlauchleitung 23 ist eine weitere Schlauchklemme 24 angeordnet. Weiterhin geht der zu der Mischeinrichtung 8 führende Schlauchleitungsabschnitt 4A nicht von dem unteren Rand, sondern von dem oberen Rand des Vollblutbeutels 3 ab. Die einander entsprechenden Teile sind mit den gleichen Bezugsziffern bezeichnet.

Die Blutverarbeitungsvorrichtung, in die das Beutelsystem von Fig. 3 eingelegt ist, arbeitet wie folgt. Im Gegensatz zu der Blutverarbeitungsvorrichtung von Fig. 1 bleibt der Buffy Coat BC nicht in dem Vollblutbeutel 3, sondern wird in den Komponentenbeutel 22 überführt. Zunächst wird die Schlauchklemme 12 geöffnet, und die Schlauchklemmen 11 und 24 werden geschlossen, wobei die Anpresseinrichtung 13 Blutplasma PLS in den Komponentenbeutel 7 drückt. Darauf hin wird die Schlauchklemme 12 geschlossen und die Schlauchklemme 24 geöffnet, wobei die Schlauchklemme 11 geschlossen bleibt. Nunmehr wird der Buffy Coat BC in den Komponentenbeutel 22 gepresst. Anschließend wird die Blutverarbeitungsvorrichtung mit geschlossenen Schlauchklemmen 12 und 24 und geöffneter Schlauchklemme 11 so betrieben, wie unter Bezugnahme auf Fig. 1 beschrieben ist.

## Patentansprüche

1. Verfahren zur Verarbeitung von Vollblut, das durch Zentrifugieren in die Blutkomponenten getrennt ist, oder von mindestens einer durch Zentrifugieren abgetrennten Blutkomponente mit folgenden Verfahrenschritten:
Bereitstellen mindestens einer Blutkomponente in einer Primärkammer,
Überführen der mindestens einen Blutkomponente aus der Primärkammer in eine Komponentenkammer, und
Mischen der mindestens einen Blutkomponente mit einer Additivlösung für die Blutkomponente,
**dadurch gekennzeichnet, dass**
die Komponentenkammer in ein erstes Kompartiment mit einem variablen Volumen für die Blutkomponente und ein zweites Kompartiment mit einem variablen Volumen für die Additivlösung getrennt wird, und
dass die Additivlösung aus dem zweiten Kompartiment gefördert und mit der mindestens einen Blutkomponente vermischt wird und die Mischung aus der Blutkomponente und der Additivlösung in das erste Kompartiment überführt wird, wobei das Volumen des ersten Kompartiments vergrößert und das Volumen des zweiten Kompartiments verkleinert wird.

2. Verfahren nach Anspruch 1, **dadurch gekennzeichnet, dass** beim Überführen der Mischung aus der Blutkomponente und der Additivlösung in das erste Kompartiment die Mischung mit einem Filter, insbesondere einem Leukozytenfilter, gefiltert wird.

3. Verfahren nach einem der Ansprüche 1 oder 2, **dadurch gekennzeichnet, dass** in der Primärkammer durch Zentrifugieren in die Blutkomponenten getrenntes Vollblut bereitgestellt wird, wobei aus der Primärkammer ein Erythrozytenkonzentrat in das erste Kompartiment der Komponentenkammer überführt wird.

4. Verfahren nach einem der Ansprüche 1 bis 3, **dadurch gekennzeichnet, dass** aus der Primärkammer vor dem Überführen des Erythrozytenkonzentrats in das erste Kompartiment der Komponentenkammer Blutplasma in einen Komponentenbeutel für Blutplasma und/oder Buffy Coat in einen Komponentenbeutel für Buffy Coat überführt wird.

5. Verfahren nach einem der Ansprüche 1 bis 4 **dadurch gekennzeichnet, dass** die Primärkammer und/oder die Komponentenkammer Beutel sind.

6. Verfahren nach Anspruch 5, **dadurch gekennzeichnet, dass** der Komponentenbeutel unter Bildung des ersten und zweiten Kompartiments innerhalb eines das erste von dem zweiten Kompartiment trennenden Bereichs zusammengedrückt wird, wobei der das erste von dem zweiten Kompartiment trennende Bereich entlang des Komponentenbeutels zur Vergrößerung des ersten Kompartiments und Verkleinerung des zweiten Kompartiments verschoben wird.

7. Verfahren nach Anspruch 5 oder 6, **dadurch gekennzeichnet, dass** auf den Primärbeutel zum Überführen einer Blutkomponente in den Komponentenbeutel eine Anpresskraft ausgeübt wird.

8. Vorrichtung zur Blutverarbeitung mit
einer Primärkammer (3) zur Aufnahme von Vollblut, das durch Zentrifugieren in Blutkomponenten getrennt ist, oder zur Aufnahme von mindestens einer durch Zentrifugieren getrennten Blutkomponente;
mindestens einer Komponentenkammer (5, 7) mit einem variablen Volumen zur Aufnahme mindestens einer Blutkomponente,
Mitteln (4, 10) zum Überführen mindestens einer Blutkomponente aus der Primärkammer in die mindestens eine Komponentenkammer, und
Mitteln (8) zum Mischen der mindestens einen Blutkomponente mit einer Additivlösung für die Blutkomponente, die einen ersten Einlass (8A) zum Zuführen der Blutkomponente und einen zweiten Einlass (8B) zum Zuführen der Additivlösung sowie einen Auslass (8C) zum Abführen der Mischung aus der Blutkomponente und der Additivlösung aufweisen,
**dadurch gekennzeichnet, dass**
die Komponentenkammer (5) einen Einlass (5A) und einen Auslass (5B) aufweist und
die Mittel (4, 10) zum Überführen einer Blutkomponente aus der Primärkammer in die Komponentenkammer aufweisen:
- eine von der Primärkammer zu dem Einlass der Mittel zum Mischen einer Blutkomponente mit einer Additivlösung führende Flüssigkeitsleitung (4A) für die Blutkomponente,
- eine von dem Auslass der Komponentenkammer zu dem zweiten Einlass der Mittel zum Mischen einer Blutkomponente mit einer Additivlösung führende Flüssigkeitsleitung (10) für die Additivlösung,
- eine von dem Auslass der Mittel zum Mischen einer Blutkomponente zu dem Einlass der Komponentenkammer führende Flüssigkeitsleitung (4B) für die Mischung aus der Blutkomponente und der Additivlösung, wobei
Mittel (14) zum Trennen der Komponentenkammer (5) in ein erstes Kompartiment (5A) mit einem variablen Volumen für die Blutkomponente und ein zweites Kompartiment (5B) mit einem variablen Volumen für die Additivlösung vorgesehen sind, die derart ausgebildet sind, dass das Volumen des ersten Kompartiments vergrößert und das Volumen des zweiten Kompartiments verkleinert werden kann.

9. Blutverarbeitungsvorrichtung nach Anspruch 8, **dadurch gekennzeichnet, dass** in der Flüssigkeitsleitung (4B) für die Mischung aus der Blutkomponente und der Additivlösung ein Filter (9), insbesondere ein Leukozytenfilter, angeordnet ist.

10. Blutverarbeitungsvorrichtung nach einem der Ansprüche 8 oder 9, **dadurch gekennzeichnet, dass** die Flüssigkeitsleitung (4) für die Blutkomponente und/oder die Flüssigkeitsleitung (10) für die Additivlösung und/oder die Flüssigkeitsleitung (6) für die Mischung aus der Blutkomponente und der Additivlösung Schlauchleitungen sind.

11. Blutverarbeitungsvorrichtung nach einem der Ansprüche 8 bis 10, **dadurch gekennzeichnet, dass** die Primärkammer (3) und/oder die Komponentenkammer (5) Beutel sind.

12. Blutverarbeitungsvorrichtung nach Anspruch 11, **dadurch gekennzeichnet, dass** die Blutverarbeitungsvorrichtung aufweist:
einen Primärbeutel (3) für Vollblut, das durch Zentrifugieren in die Blutkomponenten getrennt ist,
einen ersten Komponentenbeutel (5), der in ein erstes Kompartiment (5D) zur Aufnahme einer Mischung aus einem Erythrozytenkonzentrat und einer Additivlösung, insbesondere einer Additivlösung für das Erythrozytenkonzentrat, und ein zweites Kompartiment (5C) zur Aufnahme der Additivlösung getrennt werden kann,
einen zweiten Komponentenbeutel (7) zur Aufnahme von Blutplasma und
einen dritten Komponentenbeutel (22) zur Aufnahme von Buffy Coat, wobei der zweite und dritte Komponentenbeutel (7, 22) mit Flüssigkeitsleitungen (6, 23) mit dem Primärbeutel (3) verbunden sind.

13. Blutverarbeitungsvorrichtung nach Anspruch 11 oder 12, **dadurch gekennzeichnet, dass** die Blutverarbeitungsvorrichtung ferner Mittel (13A, 13B) zum Ausüben einer Anpresskraft auf den Primärbeutel aufweist.

14. Blutverarbeitungsvorrichtung nach einem der Ansprüche 11 bis 13, **dadurch gekennzeichnet, dass** die Mittel (14) zum Trennen des Komponentenbeutels derart ausgebildet sind, dass der Komponentenbeutel (5) unter Bildung des ersten und zweiten Kompartiments (5D, 5C) innerhalb eines das erste von dem zweiten Kompartiments trennenden Bereichs zusammengedrückt werden kann, wobei der der das erste von dem zweiten Kompartiment trennende Bereich entlang des Komponentenbeutels verschoben werden kann.

15. Blutverarbeitungsvorrichtung nach einem der Ansprüche 11 bis 14, **dadurch gekennzeichnet, dass** die Mittel (14) zum Trennen des Komponentenbeutels eine ebene ortsfeste Anpressplatte (14A) und einen entlang einer Linie einen Anpressdruck ausübenden Anpresskörper (14B, 14B') aufweisen, der in einer zu der Ebene der Anpressplatte parallelen Ebene verschiebbar geführt ist, wobei Anpresskörper und Anpressplatte unter Zwischenlage des Komponentenbeutels (5) im Abstand zueinander angeordnet sind.

16. Blutverarbeitungsvorrichtung Anspruch 15, **dadurch gekennzeichnet, dass** der Anpresskörper ein drehbar gelagerter zylindrischer Körper (14B) ist.

17. Beutelsystem für eine Blutverarbeitungsvorrichtung nach einem der Ansprüche 8 bis 16 mit
einem Primärbeutel (3) zur Aufnahme von Vollblut, das durch Zentrifugieren in die Blutkomponenten getrennt ist, oder zur Aufnahme von mindestens einer durch Zentrifugieren getrennten Blutkomponente;
mindestens einen Komponentenbeutel (5) zur Aufnahme mindestens einer Blutkomponente,
Mitteln (4, 10) zum ÜberfUhren der mindestens einen Blutkomponente aus dem Primärbeutel in den mindestens einen Komponentenbeutel und
Mitteln (8) zum Mischen der mindestens einen Blutkomponente mit einer Additivlösung für die Blutkomponente, die einen ersten Einlass (8A) zum Zuführen der Blutkomponente und einen zweiten Einlass (8B) zum Zuführen der Additivlösung sowie einen Auslass (8C) zum Abführen der Mischung aus der Blutkomponente und der Additivlösung aufweist,
**dadurch gekennzeichnet, dass** der Komponentenbeutel (5) einen Einlass (5A) und einen Auslass (5B) aufweist und
dass die Mittel (4, 10) zum Überführen einer Blutkomponente aus dem Primärbeutel in die Komponentenkammer aufweisen:
eine von dem Primärbeutel zu dem ersten Einlass der Mittel zum Mischen einer Blutkomponente mit der Additivlösung führende Schlauchleitung (4A) für die Blutkomponente,
eine von dem Auslass des Komponentbeutels zu dem zweiten Einlass der Mittel zum Mischen der Blutkomponente mit der Additivlösung führende Schlauchleitung (10) für die Additivlösung und
eine von dem Auslass der Mittel zum Mischen der Blutkomponente zu dem Einlass des Komponentenbeutels führende Schlauchleitung (4B) für die Mischung aus der Blutkomponente und der Additivlösung, wobei der Komponentenbeutel durch Mittel (14) zum Trennen der Komponentenkammer (5) in ein erstes Kompartiment (5 A) mit einem variablen Volumen für die Blutkomponente und ein zweites Kompartiment (5 B) mit einem variablen Volumen für die Additivlösung trennbar ist, derart, dass das Volumen des ersten Kompartiments durch die Mittel (14) vergrösserbar und das Volumen des zweiten Kompartiments verkleinerbar ist.

18. Beutelsystem für eine Blutverarbeitungsvorrichtung nach Anspruch 17, **dadurch gekennzeichnet, dass** in der Flüssigkeitsleitung (4B) für die Mischung aus der Blutkomponente und der Additivlösung ein Filter (9), insbesondere ein Leukozytenfilter, angeordnet ist.

19. Beutelsystem für eine Blutverarbeitungsvorrichtung nach Anspruch 17 oder 18, **dadurch gekennzeichnet, dass** das Beutelsystem aufweist: einen zweiten Komponentenbeutel (7) zur Aufnahme von Blutplasma und einen dritten Komponentenbeutel (22) zur Aufnahme von Buffy Coat, wobei der zweite und dritte Komponentenbeutel mit Schlauchleitungen (6, 23) mit dem Primärbeutel verbunden sind.

## Claims

1. Method for the processing of whole blood separated by centrifugation into the blood components or of at least one blood component separated off by centrifugation, comprising the following method steps:
providing at least one blood component in a primary chamber,
transferring the at least one blood component from the primary chamber to a component chamber, and
mixing the at least one blood component with an additive solution for the blood component,
**characterized in that**
the component chamber is separated into a first compartment having a variable volume for the blood component and a second compartment having a variable volume for the additive solution, and
that the additive solution is conveyed from the second compartment and mixed with the at least one blood component and the mixture of the blood component and the additive solution is transferred to the first compartment, wherein the volume of the first compartment is increased and the volume of the second compartment is reduced.

2. Method according to Claim 1, **characterized in that**, during the transfer of the mixture of the blood component and the additive solution to the first compartment, the mixture is filtered using a filter, more particularly a leukocyte filter.

3. Method according to either of Claims 1 and 2, **characterized in that** whole blood separated by centrifugation into the blood components is provided in the primary chamber, wherein an erythrocyte concentrate is transferred from the primary chamber to the first compartment of the component chamber.

4. Method according to any of Claims 1 to 3, **characterized in that**, before the transfer of the erythrocyte concentrate to the first compartment of the component chamber, blood plasma is transferred from the primary chamber to a component bag for blood plasma and/or buffy coat is transferred from the primary chamber to a component bag for buffy coat.

5. Method according to any of Claims 1 to 4, **characterized in that** the primary chamber and/or the component chamber are bags.

6. Method according to Claim 5, **characterized in that** the component bag is compressed within a region separating the first compartment from the second compartment to form the first and the second compartment, wherein the region separating the first compartment from the second compartment is shifted along the component bag in order to make the first compartment larger and make the second compartment smaller.

7. Method according to Claim 5 or 6, **characterized in that** pressing force is exerted on the primary bag in order to transfer a blood component to the component bag.

8. Device for blood processing, comprising
a primary chamber (3) for accommodating whole blood separated by centrifugation into blood components or for accommodating at least one blood component separated by centrifugation,
at least one component chamber (5, 7) having a variable volume for accommodating at least one blood component,
means (4, 10) for transferring at least one blood component from the primary chamber to the at least one component chamber, and
means (8) for mixing the at least one blood component with an additive solution for the blood component, which means have a first inlet (8A) for feeding the blood component and a second inlet (8B) for feeding the additive solution and also an outlet (8C) for discharging the mixture of the blood component and the additive solution,
**characterized in that**
the component chamber (5) has an inlet (5A) and an outlet (5B) and
the means (4, 10) for transferring a blood component from the primary chamber to the component chamber have:
- a liquid line (4A) for the blood component that leads from the primary chamber to the inlet of the means for mixing a blood component with an additive solution,
- a liquid line (10) for the additive solution that leads from the outlet of the component chamber to the second inlet of the means for mixing a blood component with an additive solution,
- a liquid line (4B) for the mixture of the blood component and the additive solution that leads from the outlet of the means for mixing a blood component to the inlet of the component chamber, wherein
means (14) for separating the component chamber (5) into a first compartment (5A) having a variable volume for the blood component and a second compartment (5B) having a variable volume for the additive solution are provided, which means are designed such that the volume of the first compartment can be increased and the volume of the second compartment can be reduced.

9. Blood processing device according to Claim 8, **characterized in that** a filter (9), more particularly a leukocyte filter, is arranged in the liquid line (4B) for the mixture of the blood component and the additive solution.

10. Blood processing device according to either of Claims 8 and 9, **characterized in that** the liquid line (4) for the blood component and/or the liquid line (10) for the additive solution and/or the liquid line (6) for the mixture of the blood component and the additive solution are tubing lines.

11. Blood processing device according to any of Claims 8 to 10, **characterized in that** the primary chamber (3) and/or the component chamber (5) are bags.

12. Blood processing device according to Claim 11, **characterized in that** the blood processing device comprises:
a primary bag (3) for whole blood separated by centrifugation into the blood components,
a first component bag (5) which can be separated into a first compartment (5D) for accommodating a mixture of an erythrocyte concentrate and an additive solution, more particularly an additive solution for the erythrocyte concentrate, and a second compartment (5C) for accommodating the additive solution,
a second component bag (7) for accommodating blood plasma and
a third component bag (22) for accommodating buffy coat, wherein the second and the third component bag (7, 22) are connected to the primary bag (3) with liquid lines (6, 23).

13. Blood processing device according to Claim 11 or 12, **characterized in that** the blood processing device further comprises means (13A, 13B) for exerting a pressing force on the primary bag.

14. Blood processing device according to any of Claims 11 to 13, **characterized in that** the means (14) for separating the component bag are designed such that the component bag (5) can be compressed within a region separating the first compartment from the second compartment to form the first and the second compartment (5D, 5C), wherein the region separating the first compartment from the second compartment can be shifted along the component bag.

15. Blood processing device according to any of Claims 11 to 14, **characterized in that** the means (14) for separating the component bag comprise a planar stationary pressing plate (14A) and a pressing body (14B, 14B') which exerts a pressing pressure along a line and which is guided in a shiftable manner in a plane parallel to the plane of the pressing plate, wherein pressing body and pressing plate are arranged at a distance from one another with interposition of the component bag (5).

16. Blood processing device according to Claim 15, **characterized in that** the pressing body is a pivoted cylindrical body (14B).

17. Bag system for a blood processing device according to any of claims 8 to 16, comprising
a primary bag (3) for accommodating whole blood separated by centrifugation into the blood components or for accommodating at least one blood component separated by centrifugation,
at least one component bag (5) for accommodating
at least one blood component,
means (4, 10) for transferring the at least one blood component from the primary bag to the at least one component bag and
means (8) for mixing the at least one blood component with an additive solution for the blood component, which means have a first inlet (8A) for feeding the blood component and a second inlet (8B) for feeding the additive solution and also an outlet (8C) for discharging the mixture of the blood component and the additive solution,
**characterized in that**
the component bag (5) has an inlet (5A) and an outlet (5B) and
that the means (4, 10) for transferring a blood component from the primary bag to the component chamber have:
a tubing line (4A) for the blood component that leads from the primary bag to the first inlet of the means for mixing a blood component with the additive solution,
a tubing line (10) for the additive solution that leads from the outlet of the component bag to the second inlet of the means for mixing the blood component with the additive solution, and
a tubing line (4B) for the mixture of the blood component and the additive solution that leads from the outlet of the means for mixing the blood component to the inlet of the component bag, wherein the component bag is separable, via means (14) for separating the component chamber (5), into a first compartment (5A) having a variable volume for the blood component and a second compartment (5B) having a variable volume for the additive solution such that the volume of the first compartment is increasable via the means (14) and the volume of the second compartment is reducible.

18. Bag system for a blood processing device according to Claim 17, **characterized in that** a filter (9), more particularly a leukocyte filter, is arranged in the liquid line (4B) for the mixture of the blood component and the additive solution.

19. Bag system for a blood processing device according to Claim 17 or 18, **characterized in that** the bag system comprises:
a second component bag (7) for accommodating blood plasma and
a third component bag (22) for accommodating buffy coat, wherein the second and the third component bag are connected to the primary bag with tubing lines (6, 23).

## Revendications

1. Procédé pour le traitement de sang complet, qui est séparé par centrifugation en ses composants sanguins, ou d'au moins un composant sanguin séparé par centrifugation, présentant les étapes de procédé suivantes :
disposition d'au moins un composant sanguin dans une chambre primaire,
transfert dudit au moins un composant sanguin de la chambre primaire dans une chambre à composant, et
mélange dudit au moins un composant sanguin avec une solution d'additif pour le composant sanguin,
**caractérisé en ce que** la chambre à composant est séparée en un premier compartiment présentant un volume variable pour le composant sanguin et en un deuxième compartiment présentant un volume variable pour la solution d'additif, et **en ce que** la solution d'additif est transportée hors du deuxième compartiment et mélangée avec ledit au moins un composant sanguin et le mélange du composant sanguin et de la solution d'additif est transféré dans le premier compartiment,
le volume du premier compartiment étant augmenté et le volume du deuxième compartiment étant diminué.

2. Procédé selon la revendication 1, **caractérisé en ce que** lors du transfert du mélange du composant sanguin et de la solution d'additif dans le premier compartiment, le mélange est filtré à l'aide d'un filtre, en particulier d'un filtre à leucocytes.

3. Procédé selon l'une quelconque des revendications 1 ou 2, **caractérisé en ce qu'**on dispose, dans la chambre primaire, du sang complet séparé par centrifugation en ses composants sanguins, un concentrat d'érythrocytes étant transféré de la chambre primaire dans le premier compartiment de la chambre à composant.

4. Procédé selon l'une quelconque des revendications 1 à 3, **caractérisé en ce qu'**avant le transfert du concentrat d'érythrocytes dans le premier compartiment de la chambre à composant, on transfère, à partir de la chambre primaire, le plasma sanguin dans une poche à composant pour plasma sanguin et/ou la couche leucocytaire (buffy coat) dans une poche à composant pour couche leucocytaire.

5. Procédé selon l'une quelconque des revendications 1 à 4, **caractérisé en ce que** la chambre primaire et/ou la chambre à composant sont des poches.

6. Procédé selon la revendication 5, **caractérisé en ce que** la poche à composant est comprimée dans une zone séparant le premier compartiment du deuxième compartiment avec formation du premier et du deuxième compartiment, la zone séparant le premier compartiment du deuxième compartiment étant déplacée le long de la poche à composant pour augmenter le premier compartiment et diminuer le deuxième compartiment.

7. Procédé selon la revendication 5 ou 6, **caractérisé en ce qu'**une pression d'appui est exercée sur la poche primaire pour transférer un composant sanguin dans la poche à composant.

8. Dispositif pour le traitement du sang présentant
une chambre primaire (3) pour recevoir du sang complet, qui est séparé par centrifugation en composants sanguins, ou pour recevoir au moins un composant sanguin séparé par centrifugation ;
au moins une chambre à composant (5, 7) présentant un volume variable pour recevoir au moins un composant sanguin,
des moyens (4, 10) pour transférer ledit au moins un composant sanguin de la chambre primaire dans ladite au moins une chambre à composant, et
des moyens (8) pour mélanger ledit au moins un composant sanguin avec une solution d'additif pour le composant sanguin, qui présentent une première entrée (8A) pour introduire le composant sanguin et une deuxième entrée (8B) pour introduire la solution d'additif ainsi qu'une sortie (8C) pour évacuer le mélange constitué par le composant sanguin et la solution d'additif, **caractérisé en ce que** la chambre à composant (5) présente une entrée (5A) et une sortie (5B), et **en ce que** les moyens (4, 10) pour transférer un composant sanguin de la chambre primaire dans la chambre à composant présentent :
- une conduite de liquide (4A) pour le composant sanguin, allant de la chambre primaire à l'entrée des moyens pour mélanger un composant sanguin avec une solution d'additif,
- une conduite de liquide (10) pour la solution d'additif, allant de la sortie de la chambre à composant à la deuxième entrée des moyens pour mélanger un composant sanguin avec une solution d'additif,
- une conduite de liquide (4B) pour le mélange du composant sanguin et de la solution d'additif, allant de la sortie des moyens pour mélanger un composant sanguin à l'entrée de la chambre à composant,
des moyens (14) étant prévus pour séparer la chambre à composant (5) en un premier compartiment (5A) présentant un volume variable pour le composant sanguin et en un deuxième compartiment (5B) présentant un volume variable pour la solution d'additif, qui sont conçus de manière telle que le volume du premier compartiment peut être augmenté et le volume du deuxième compartiment peut être diminué.

9. Dispositif pour le traitement du sang selon la revendication 8, **caractérisé en ce qu'**un filtre (9), en particulier un filtre à leucocytes, est disposé dans la conduite de liquide (4B) pour le mélange du composant sanguin et de la solution d'additif.

10. Dispositif pour le traitement du sang selon l'une quelconque des revendications 8 ou 9, **caractérisé en ce que** la conduite de liquide (4) pour le composant sanguin et/ou la conduite de liquide (10) pour la solution d'additif et/ou la conduite de liquide (6) pour le mélange du composant sanguin et de la solution d'additif sont des conduites souples.

11. Dispositif pour le traitement du sang selon l'une quelconque des revendications 8 à 10, **caractérisé en ce que** la chambre primaire (3) et/ou la chambre à composant (5) sont des poches.

12. Dispositif pour le traitement du sang selon la revendication 11, **caractérisé en ce que** le dispositif pour le traitement du sang présente :
une poche primaire (3) pour le sang complet, qui est séparé par centrifugation en ses composants sanguins,
une première poche à composant (5), qui peut être séparée en un premier compartiment (5D) pour recevoir un mélange d'un concentrat d'érythrocytes et d'une solution d'additif, en particulier d'une solution d'additif pour le concentrat d'érythrocytes, et en un deuxième compartiment (5C) pour recevoir la solution d'additif,
une deuxième poche à composant (7) pour recevoir du plasma sanguin et
une troisième poche à composant (22) pour recevoir la couche leucocytaire, la deuxième et la troisième poche à composant (7, 22) étant reliées via des conduites de liquide (6, 23) à la poche primaire (3).

13. Dispositif pour le traitement du sang selon la revendication 11 ou 12, **caractérisé en ce que** le dispositif pour le traitement du sang présente en outre des moyens (13A, 13B) pour exercer une pression d'appui sur la poche primaire.

14. Dispositif pour le traitement du sang selon l'une quelconque des revendications 11 à 13, **caractérisé en ce que** les moyens (14) pour séparer la poche à composant sont conçus de manière telle que la poche à composant (5) peut être comprimée, avec formation du premier et du deuxième compartiment (5D, 5C), dans une zone séparant le premier du deuxième compartiment, la zone séparant le premier du deuxième compartiment pouvant être déplacée le long de la poche à composant.

15. Dispositif pour le traitement du sang selon l'une quelconque des revendications 11 à 14, **caractérisé en ce que** les moyens (14) pour séparer la poche à composant présentent une plaque d'appui (14A) plane fixe et un corps d'appui (14B, 14B') exerçant une pression d'appui le long d'une ligne, qui est guidé de manière mobile dans un plan parallèle au plan de la plaque d'appui, le corps d'appui et la plaque d'appui étant disposés à une certaine distance l'un de l'autre avec intercalation de la poche à composant (5).

16. Dispositif pour le traitement du sang selon la revendication 15, **caractérisé en ce que** le corps d'appui est un corps cylindrique (14B) logé de manière rotative.

17. Système à poche pour un dispositif pour le traitement du sang selon l'une quelconque des revendications 8 à 16, présentant
une poche primaire (3) pour recevoir du sang complet, qui est séparé par centrifugation en ses composants sanguins, ou pour recevoir au moins un composant sanguin séparé par centrifugation ;
au moins une poche à composant (5) pour recevoir au moins un composant sanguin,
des moyens (4, 10) pour transférer ledit au moins un composant sanguin de la poche primaire dans ladite au moins une poche à composant, et
des moyens (8) pour mélanger ledit au moins un composant sanguin avec une solution d'additif pour le composant sanguin, qui présentent une première entrée (8A) pour introduire le composant sanguin et une deuxième entrée (8B) pour introduire la solution d'additif ainsi qu'une sortie (8C) pour évacuer le mélange constitué par le composant sanguin et la solution d'additif, **caractérisé en ce que** la poche à composant (5) présente une entrée (5A) et une sortie (5B) et **en ce que** les moyens (4, 10) pour transférer un composant sanguin de la poche primaire dans la chambre à composant présentent :
une conduite souple (4A) pour le composant sanguin, allant de la poche primaire à la première entrée des moyens pour mélanger un composant sanguin avec la solution d'additif,
une conduite souple (10) pour la solution d'additif, allant de la sortie de la poche à composant à la deuxième entrée des moyens pour mélanger le composant sanguin avec la solution d'additif,
une conduite souple (4B) pour le mélange du composant sanguin et de la solution d'additif, allant de la sortie des moyens pour mélanger le composant sanguin à l'entrée de la poche à composant, la poche à composant pouvant être séparée par des moyens (14) pour séparer la chambre à composant (5) en un premier compartiment (5A) présentant un volume variable pour le composant sanguin et en un deuxième compartiment (5B) présentant un volume variable pour la solution d'additif, de manière telle que le volume du premier compartiment peut être augmenté et le volume du deuxième compartiment peut être diminué par les moyens (14).

18. Système à poche pour le dispositif pour le traitement du sang selon la revendication 17, **caractérisé en ce qu'**un filtre (9), en particulier un filtre à leucocytes, est disposé dans la conduite de liquide (4B) pour le mélange du composant sanguin et de la solution d'additif.

19. Système à poche pour le dispositif pour le traitement du sang selon la revendication 17 ou 18, **caractérisé en ce que** le système à poche présente :
une deuxième poche à composant (7) pour recevoir du plasma sanguin et
une troisième poche à composant (22) pour recevoir la couche leucocytaire, la deuxième et la troisième poche à composant étant reliées via des conduites souples (6, 23) à la poche primaire.
